# EUROPÄISCHE PATENTANMELDUNG

(11) **EP 2 881 126 A1**
(43) Veröffentlichungstag der Anmeldung: **10.06.2015**
(21) Anmeldenummer: 14196873.5
(22) Anmeldetag: 09.12.2014
(51) Int. Cl.: A61L 9/20, F21S 8/04, F21S 8/02, E04B 9/00

(54) **Multifunktionale Deckenleuchte**

(30) Priorität: 09.12.2013 DE 102013225255
(71) Anmelder: CleanTec Lighting GmbH, 04316 Leipzig (DE)
(72) Erfinder: Schuck, Frank, 04316 Leipzig (DE); Zimmermann, Jürgen, 04316 Leipzig (DE); Wipprich, Carola, 98544 Zella-Mehlis (DE); Wipprich, Walter, 98544 Zella-Mehlis (DE)
(74) Vertreter: Platzöder, Michael Christian

(57) **Zusammenfassung**

Eine multifunktionale Deckenleuchte (16) weist eine Beleuchtungsvorrichtung (24), welche ein Leuchtengehäuse (25) und wenigstens ein Leuchtmittel zum Emittieren von Licht im sichtbaren Wellenlängenbereich in dem Leuchtengehäuse aufweist; eine Luftreinigungsvorrichtung (30), welche einen Strömungskasten (31) aufweist, welcher auf einer der Raumdecke (11) zugewandten Seite des Leuchtengehäuses (24) angeordnet ist und einen Luftkanal (32) darin definiert; und eine Befestigungseinrichtung (26) zum Montieren der Beleuchtungsvorrichtung (24) und der Luftreinigungsvorrichtung (30) in einer von einer Raumdecke (11) eines Raums (10) abgehängten Decke (12) auf. Ein Lufteinlass (33) des Strömungskastens (31) steht über eine erste Öffnung (20) mit dem Raum (10) in Verbindung und ein Luftauslass (35) des Strömungskastens (31) steht über eine zweite Öffnung (22) mit dem Raum (10) in Verbindung, und in dem Strömungskasten (31) ist in Gebläse (38) angeordnet, um Luft vom Lufteinlass (33) durch den Luftkanal (32) hindurch zum Luftauslass (35) des Strömungskastens (31) zu fördern. Die Luftreinigungsvorrichtung (30) weist eine UV-Strahlungseinrichtung (46) auf, welche ausgestaltet ist, um die durch den Luftkanal (32) des Strömungskastens (31) hindurch strömende Luft (40) mittels UV-Strahlung zu reinigen.

## Beschreibung

Die Erfindung betrifft eine multifunktionale Deckenleuchte, insbesondere eine in eine abgehängte Decke integrierbare, multifunktionale Deckenleuchte.

Von Raumdecken abgehängte Decken sind in vielfältigen Ausgestaltungen bekannt. Eine weit verbreitete Art von abgehängten Decken sind die so genannten Kassettendecken, bei denen eine Vielzahl von meist quadratischen Deckenelementen an einer Unterkonstruktion montiert ist. Neben Deckenelementen mit zum Beispiel Design- und/oder Schalldämpfungseigenschaften sind auch Deckenleuchten bekannt, die in derartige Kassettendecken eingebaut werden können.

Neben der Beleuchtung eines Raums werden häufig auch eine Klimatisierung und/oder eine Reinigung des Raums bzw. der Raumluft gefordert. Die DE 20 2013 000 809 U1 offenbart eine Ultraviolett-Luftdesinfektionsvorrichtung zur Desinfektion von Raumluft, welche neben einer Desinfektionseinheit mit einem UV-Strahler und einer aktiven Luftführungseinrichtung eine Beleuchtungseinheit mit einem Leuchtmittel zum Emittieren von Licht im sichtbaren Wellenlängenbereich aufweist. Diese UV-Luftdesinfektionsvorrichtung kann als Hängevorrichtung zur Anbringung an einer Raumdecke ausgebildet sein.

Es ist die Aufgabe der Erfindung, eine verbesserte Deckenleuchte zu schaffen, welche in eine abgehängte Decke integriert werden kann und neben einer Raumbeleuchtung auch die Funktionalität einer Luftreinigung bereitstellt.

Diese Aufgabe wird gelöst durch eine Deckenleuchte mit den Merkmalen des Anspruchs 1. Besonders bevorzugte Ausgestaltungen und Weiterbildungen der Erfindung sind in den abhängigen Ansprüchen angegeben.

Die Deckenleuchte der Erfindung weist auf: eine Beleuchtungsvorrichtung, welche ein Leuchtengehäuse und wenigstens ein Leuchtmittel zum Emittieren von Licht im sichtbaren Wellenlängenbereich in dem Leuchtengehäuse aufweist; eine Luftreinigungsvorrichtung, welche einen Strömungskasten aufweist, welcher auf einer der Raumdecke zugewandten Seite des Leuchtengehäuses angeordnet ist und einen Luftkanal darin definiert; und eine Befestigungseinrichtung zum Montieren der Beleuchtungsvorrichtung und der Luftreinigungsvorrichtung in einer von einer Raumdecke eines Raums abgehängten Decke. Dabei steht ein Lufteinlass des Strömungskastens über eine erste Öffnung mit dem Raum in Verbindung und steht ein Luftauslass des Strömungskastens über eine zweite Öffnung mit dem Raum in Verbindung, und ist in dem Strömungskasten ein Gebläse angeordnet, um Luft vom Lufteinlass durch den Luftkanal hindurch zum Luftauslass des Strömungskastens zu fördern. Außerdem weist die Luftreinigungsvorrichtung eine UV-Strahlungseinrichtung auf, welche ausgestaltet ist, um die durch den Luftkanal des Strömungskastens hindurch strömende Luft mittels UV-Strahlung zu reinigen.

Eine solche multifunktionale Deckenleuchte ist mit Hilfe der Befestigungseinrichtung in eine von einer Raumdecke abgehängte Decke integrierbar. Besonders bevorzugt ist die Deckenleuchte der Erfindung in eine abgehängte Kassettendecke integrierbar. Die Deckenleuchte weist vorzugsweise zu Standardmaßen von 600 mm × 600 mm oder 620 mm × 620 mm kompatible Abmessungen auf, sodass sie ohne zusätzlichen Anpassungsaufwand in Standard-Kassettendecken eingebaut werden kann.

Der Begriff "Leuchtengehäuse" soll in diesem Zusammenhang jede Art von Konstruktion bezeichnen, die geeignet ist, wenigstens ein Leuchtmittel an einer vorbestimmten Position zu halten. Das Leuchtengehäuse kann ein vollständig bzw. allseitig geschlossenes Gehäuse, ein teils geschlossenes und teils offenes Gehäuse oder eine offene Rahmenkonstruktion sein. Das wenigstens eine Leuchtmittel in dem Leuchtengehäuse ist an dem Leuchtengehäuse vorzugsweise so montiert, dass es zumindest teilweise in seinem Inneren aufgenommen ist. Das Leuchtengehäuse weist vorzugsweise mechanische und elektrische Anschlusseinrichtungen für das wenigstens eine Leuchtmittel auf, welche innerhalb und/oder außerhalb des Leuchtengehäuses platziert sein können. Das Leuchtengehäuse hat eine Leuchtfläche, durch welche das von dem wenigstens einen Leuchtmittel erzeugte Licht in den Raum ausgesendet wird. Diese Leuchtfläche ist zusammenhängend oder abschnittweise ausgebildet, sie hat vorzugsweise eine quadratische, rechteckige oder kreisförmige Grundform. Die Leuchtfläche kann offen ausgestaltet sein, eine transparente oder semitransparente Abdeckung aufweisen, mit Reflektoren versehen sein, Lichtleitstrukturen aufweisen und dergleichen.

Neben der Raumbeleuchtung erfüllt die multifunktionale Deckenleuchte der Erfindung zudem den Zweck einer Reinigung bzw. Desinfektion der Raumluft. Durch die Anordnung des Strömungskastens der Luftreinigungsvorrichtung auf der der Raumdecke zugewandten Seite des Leuchtengehäuses ist neben der Beleuchtungsvorrichtung auch die Luftreinigungsvorrichtung der Deckenleuchte in eine abgehängte (Kassetten-)Decke integrierbar. Dabei ist die UV-Strahlungseinrichtung durch das Leuchtengehäuse abgeschirmt und es sind keine zusätzlichen Maßnahmen erforderlich, um einen UV-Strahlungsaustritt in den Raum zu verhindern. Das Design und die Beleuchtungswirkung der Deckenleuchte werden durch die Luftreinigungsvorrichtung aufgrund dieser Anordnung oberhalb der Beleuchtungsvorrichtung nicht beeinträchtigt.

Vorzugsweise ist die Luftreinigungsvorrichtung derart dimensioniert, dass in etwa ein Luftvolumen des Raums gereinigt bzw. rein gehalten werden kann, das dem durch die Beleuchtungsvorrichtung beleuchten Raumvolumen entspricht. Auf diese Weise kann erreicht werden, dass bei einer Bestückung der abgehängten Decke mit Deckenleuchten der Erfindung zum Zwecke einer ausreichenden Raumbeleuchtung automatisch auch eine ausreichende Luftreinigungswirkung für den Raum vorhanden ist.

Die spezielle Kombination aus Beleuchtungsvorrichtung, Luftreinigungsvorrichtung und Befestigungseinrichtung ermöglicht bei der erfindungsgemäßen Deckenleuchte einen kompakten Aufbau und eine kompakte Montage in einer abgehängten Decke.

Der Begriff "Befestigungseinrichtung" soll in diesem Zusammenhang jede Art von Einrichtung oder Maßnahme bezeichnen, die geeignet ist, die Komponenten der Deckenleuchte dauerhaft oder vorzugsweise lösbar in einer abgehängten Decke in einem Raum zu montieren. Die Beleuchtungsvorrichtung und die Luftreinigungsvorrichtung können dabei mit Hilfe der Befestigungseinrichtung gemeinsam bzw. als eine Baueinheit oder separat bzw. als Einzelkomponenten in der abgehängten Decke montierbar sein. Vorzugsweise ist die Luftreinigungsvorrichtung fest (dauerhaft oder lösbar) mit der Beleuchtungsvorrichtung verbunden, sodass sie als eine Baueinheit gemeinsam in der abgehängten Decke montiert werden können.

Die Beleuchtung des Raums wird durch die in die Deckenleuchte integrierte Luftreinigungsvorrichtung nicht gestört. Da die UV-Strahlungseinrichtung die UV-Strahlung nicht in den Raum aussendet, wird die Luft mittels eines Gebläses durch den Luftkanal und somit durch den Wirkungsbereich der UV-Strahlungseinrichtung gefördert. Der Begriff Gebläse umfasst in diesem Zusammenhang jede Art von Einrichtung, die geeignet ist, Luft durch einen Kanal zu fördern; hierzu zählen insbesondere Gebläse, Lüfter, Ventilatoren und dergleichen.

Die UV-Strahlungseinrichtung der Luftreinigungsvorrichtung ermöglicht eine sehr effiziente Reinigung und Desinfektion der Raumluft, da die UV-Strahlung Keime, Bakterien, Viren, Hefen, Schimmelpilzsporen und dergleichen in der Raumluft abtöten kann. Die UV-Strahlungseinrichtung emittiert UV-Strahlung (100 - 380 nm), insbesondere kurzwellige UV-C-Strahlung (100 - 280 nm). Die von der UV-Strahlungsvorrichtung erzeugte UV-C-Strahlung kann dabei im keimtötenden Bereich von etwa 200 - 280 nm (Leistungsmaximum bevorzugt bei etwa 254 nm) und/oder im ozonbildenden Bereich von etwa 100 - 200 nm liegen. Die desinfizierende Wirkung von UV-Strahlung, insbesondere UV-C-Strahlung ist hinlänglich bekannt und muss deshalb an dieser Stelle nicht weiter erörtert werden.

Die erste Öffnung und die zweite Öffnung sind vorzugsweise möglichst weit voneinander beabstandet, sodass die über die zweite Öffnung aus dem Luftkanal des Strömungskastens ausgegebene, gereinigte Luft nicht sofort wieder über die erste Öffnung in den Luftkanal gesaugt wird. Die erste und die zweite Öffnung haben vorzugsweise einen im Wesentlichen gleich großen Strömungsquerschnitt, bevorzugt auch eine im Wesentlichen gleiche Querschnittsform. Die erste und/oder die zweite Öffnung sind vorzugsweise schlitz- oder langlochförmig ausgestaltet.

In einer bevorzugten Ausgestaltung der Erfindung sind die erste Öffnung und/oder die zweite Öffnung in Ausnehmungen des Leuchtengehäuses positioniert oder in dem Leuchtengehäuse selbst ausgebildet. Diese Ausgestaltung hat den Vorteil, dass das Leuchtengehäuse den gesamten (Kassetten-)Abschnitt der Deckenleuchte überdecken kann, ohne dass zusätzliche Luftkanalöffnungen in der Decke vorgesehen werden müssen.

Bei dieser Ausgestaltung ist der Lufteinlass des Strömungskastens vorzugsweise mit der ersten Öffnung über ein erstes Luftleitelement verbunden und/oder ist der Luftauslass des Strömungskastens vorzugsweise mit der zweiten Öffnung über ein zweites Luftleitelement verbunden. Die Luftleitelemente sind vorzugsweise derart ausgebildet, dass sie einen möglichst geringen Strömungswiderstand besitzen und die Luft möglichst ohne Verluste des Strömungsvolumens leiten können. Die Luftleitelemente definieren vorzugsweise Luftkanäle in ihrem Inneren, welche geradlinig oder ein- oder mehrfach gebogen oder gekrümmt zwischen den Öffnungen und dem Luftkanal des Strömungskastens verlaufen. In einer bevorzugten Ausführungsvariante sind die erste und die zweite Öffnung im ersten bzw. zweiten Luftleitelement ausgebildet. Über die Luftleitelemente kann der Strömungskasten der Luftreinigungsvorrichtung variabel an die Leuchtengehäuse von verschieden ausgestalteten oder dimensionierten Beleuchtungsvorrichtungen angeschlossen werden. Für verschiedene Deckenleuchten müssen somit nicht alle Komponenten der Deckenleuchte modifiziert werden.

In einer bevorzugten Ausgestaltung der Erfindung ist im Luftkanal des Strömungskastens zwischen dem Lufteinlass und dem Luftauslass ein Luftfilter angeordnet. Der Luftfilter ist vorzugsweise stromauf des Gebläses und/oder stromauf der UV-Strahlungseinrichtung positioniert. Der Luftfilter dient einer zusätzlichen Reinigung der Raumluft und reduziert eine Verschmutzung des Luftkanals, des Gebläses und/oder der UV-Strahlungseinrichtung. In einer anderen bevorzugten Ausgestaltung der Erfindung ist ein solcher Luftfilter im ersten oder zweiten Luftleitelement oder in der ersten oder zweiten Öffnung angeordnet.

In einer weiteren bevorzugten Ausgestaltung der Erfindung ist die UV-Strahlungseinrichtung unabhängig von dem wenigstens einen Leuchtmittel der Beleuchtungsvorrichtung betreibbar. Allgemeiner ist die Luftreinigungsvorrichtung vorzugsweise unabhängig von der Beleuchtungsvorrichtung betreibbar. Hierdurch besteht insbesondere die Möglichkeit, eine Reinigung der Raumluft auch bei ausgeschalteter Raumbeleuchtung durchzuführen; andererseits kann auch die Raumbeleuchtung ohne gleichzeitige Luftreinigung erfolgen.

In einer weiteren bevorzugten Ausgestaltung der Erfindung weist die Beleuchtungsvorrichtung einen Kühlkörper und/oder eine Steuervorrichtung auf, welche(r) zumindest teilweise im Luftkanal des Strömungskastens angeordnet ist/sind. Die durch den Luftkanal geförderte Raumluft kann so gleichzeitig zum Kühlen von Leistungsbauteilen, etc. der Beleuchtungsvorrichtung genutzt werden. Vorzugsweise können zusätzliche Maßnahmen zur Kühlung entfallen oder zumindest reduziert werden.

In einer weiteren bevorzugten Ausgestaltung der Erfindung weist die Luftreinigungsvorrichtung einen Kühlkörper und/oder eine Steuervorrichtung und/oder einen Gebläsemotor auf, welche(r) zumindest teilweise im Luftkanal des Strömungskastens angeordnet ist/sind. Strömungskastens angeordnet ist/sind. Die durch den Luftkanal geförderte Raumluft kann so gleichzeitig zum Kühlen von Leistungsbauteilen, etc. der Luftreinigungsvorrichtung genutzt werden. Vorzugsweise können zusätzliche Maßnahmen zur Kühlung entfallen oder zumindest reduziert werden.

In einer noch weiteren bevorzugten Ausgestaltung der Erfindung ist die Befestigungseinrichtung integral mit dem Leuchtengehäuse der Beleuchtungsvorrichtung oder dem Strömungskasten der Luftreinigungsvorrichtung ausgebildet. Diese Ausgestaltung ermöglicht eine kompakte Bauweise und eine einfache Montage der Deckenleuchte. Die Befestigungseinrichtung ist beispielsweise in Form eines an dem Leuchtengehäuse angeformten Halterahmens ausgestaltet.

Vorzugsweise ist der Strömungskasten der Luftreinigungsvorrichtung fest (lösbar oder dauerhaft) mit dem Leuchtengehäuse der Beleuchtungsvorrichtung verbunden, sodass beide Komponenten gemeinsam in der abgehängten Decke montiert werden können. Insbesondere können auf diese Weise beide Komponenten mit Hilfe einer gemeinsamen, einfach ausgestalteten Befestigungseinrichtung in der abgehängten Decke montiert werden.

In einer weiteren bevorzugten Ausgestaltung der Erfindung ist die UV-Strahlungseinrichtung mit einem Lichtaustritt gekoppelt, um von der UV-Strahlungseinrichtung erzeugte Strahlung in den Raum auszusenden. Dabei ist im entsprechenden Strahlengang vorzugsweise ein UV-Filter vorgesehen, sodass keine schädlichen Strahlungsanteile in den Raum austreten können. Bei dieser Ausgestaltung kann die Strahlung der UV-Strahlungseinrichtung (insbesondere deren unschädliche UV-Anteile und die sichtbaren Lichtanteile) mit zur Raumbeleuchtung genutzt werden. Die Energieeffizienz der Deckenleuchte kann so verbessert werden.

In einer weiteren bevorzugten Ausgestaltung der Erfindung weist das wenigstens eine Leuchtmittel der Beleuchtungsvorrichtung wenigstens eine Leuchtiode oder Leuchtdiodengruppe auf. Durch die Verwendung von Leuchtdioden(gruppen) kann die Energieeffizienz der Deckenleuchte der Erfindung verbessert werden.

Die Deckenleuchte der Erfindung ist besonders von Vorteil in Räumen, in denen eine saubere oder sogar möglichst keimfreie Raumluft wünschenswert ist. Zu diesen Räumen zählen insbesondere Räume im Gesundheitswesen (z.B. Arztpraxen, Krankenhäuser, Behandlungsräume, Wartezimmer für Patienten, etc.), aber auch Räume mit viel Publikumsverkehr (z.B. Verkaufsräume von Geschäften und Kaufhäusern, etc.), Räume des öffentlichen Nah- und Fernverkehrs (z.B. Fahrzeuge, Wartehallen, etc.), aber auch privat oder geschäftlich genutzte Räume (z.B. Großraumbüros, Besprechungszimmer, Werkhallen, etc.).

Obige sowie weitere Merkmale und Vorteile der Erfindung werden aus der nachfolgenden Beschreibung von bevorzugten, nicht-einschränkenden Ausführungsbeispielen anhand der beiliegenden Zeichnungen besser verständlich. Darin zeigen, zum Teil schematisch:
- Fig. 1: eine perspektivische Teilansicht eines Raums mit einer abgehängten Decke;
- Fig. 2: eine Perspektivansicht (von schräg oben) einer Deckenleuchte gemäß einem Ausführungsbeispiel der Erfindung (mit einem vom Strömungskasten abgenommenem Deckel);
- Fig. 3: eine Perspektivansicht (von schräg unten) eines Leuchtengehäuses der Deckenleuchte von Fig. 2;
- Fig. 4: eine Perspektivansicht des Strömungskastens der Deckenleuchte von Fig. 2 (ohne Deckel und ohne UV-Strahlungseinrichtung); und
- Fig. 5: eine Schnittansicht der Luftreinigungsvorrichtung der Deckenleuchte von Fig. 2.

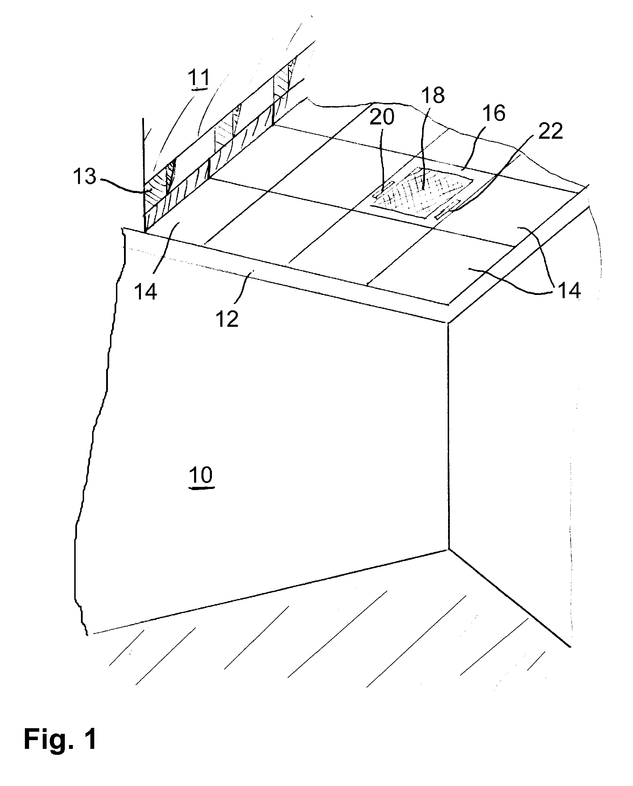

Fig. 1 zeigt in stark vereinfachter Darstellungsweise einen Raum 10 mit einer von einer Raumdecke 11 abgehängten (Kassetten-)Decke 12. Die Kassettendecke 12 ist über eine Unterkonstruktion 13 an der Raumdecke 11 angebracht.

Die abgehängte Kassettendecke 12 enthält eine Vielzahl von Deckenelementen 14. Die Deckenelemente 14 haben einen im Wesentlichen quadratischen (wahlweise auch rechteckigen) Grundriss von zum Beispiel etwa 600 mm × 600 mm oder 620 mm × 620 mm. Die Deckenelemente 14 haben insbesondere Design- und/oder Schalldämpfungsfunktionen.

Ein Teil der Deckenelemente 14 ist durch Deckenleuchten 16 ersetzt. Die Deckenleuchten 16 weisen an ihrer dem Raum zugewandten Unterseite eine (durchgängige oder unterbrochene) Leuchtfläche 18 auf, durch welche sie Licht im sichtbaren Wellenlängenbereich (380 nm - 780 nm) in den Raum 10 aussenden. In einer Ausführungsvariante wird von den Deckenleuchten 16 sonnenlichtähnliches Licht emittiert (sog. Tageslichtleuchten).

Die Unterseiten der Deckenleuchten 16 sind vorzugsweise im Wesentlichen fluchtend zu den Unterseiten der Deckenelemente 14 ausgerichtet. Wie in Fig. 1 angedeutet, weisen die Deckenleuchten 16 an ihrer Unterseite zudem eine erste zum Beispiel schlitzartige Öffnung 20 und eine zweite zum Beispiel schlitzartige Öffnung 22 auf, deren Funktion unten noch näher erläutert wird.

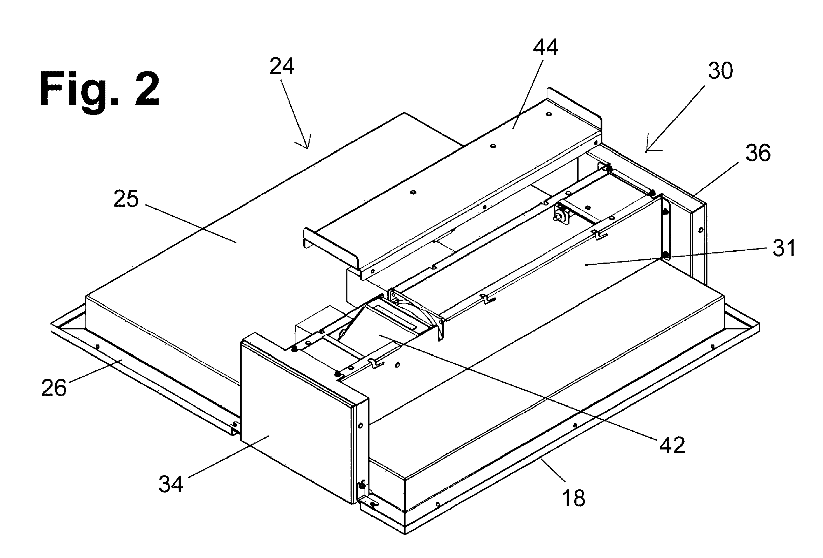

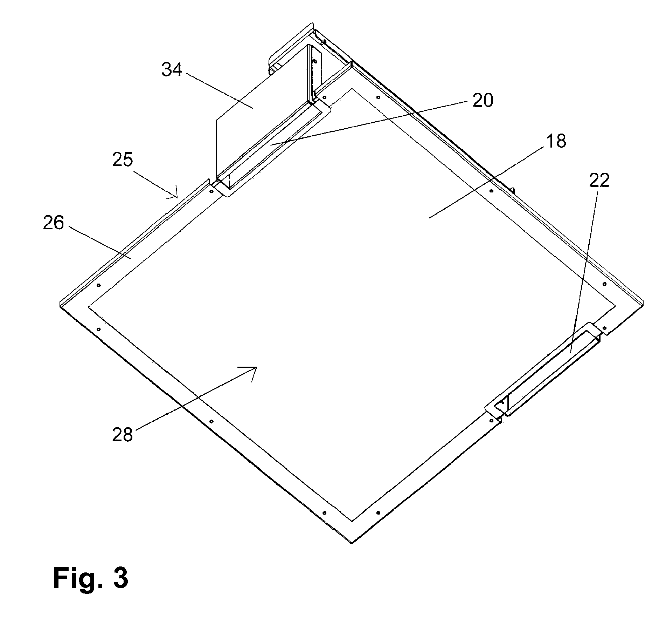

Wie in Fig. 2 dargestellt, weist die Deckenleuchte 16 an ihrer dem Raum 10 zugewandten Unterseite eine Beleuchtungsvorrichtung 24 auf. Wie in Fig. 3 dargestellt, weist diese Beleuchtungsvorrichtung 24 zum Beispiel ein Leuchtengehäuse 25 mit einem umlaufenden Rand 26, der als eine Befestigungseinrichtung der Erfindung dient, und einer Ausnehmung 28 für Leuchtmittel auf. Bei den nicht dargestellten Leuchtmitteln handelt es sich beispielsweise um Leuchtdioden oder Leuchtdiodengruppen. Die Beleuchtungsvorrichtung 24 weist optional zudem eine Abdeckung zum Beispiel aus einem (semi-)transparenten Kunststoffmaterial (beispielsweise Acryl) zum Verschließen der Ausnehmung 28 und/oder andere Lichtleitstrukturen, Reflektoren, Jalousien, etc. auf, welche in Fig. 3 weggelassen sind.

Wie in Fig. 3 dargestellt, sind die erste Öffnung 20 und die zweite Öffnung 22 in einem ersten bzw. zweiten Luftleitelement 34, 36 ausgebildet und in entsprechenden Ausnehmungen im Rand 26 des Leuchtengehäuses 25 positioniert. Außerdem ist der als Befestigungseinrichtung dienende Rand 26 integral mit dem Leuchtengehäuse 25 ausgebildet. Über den Rand 26 können die Beleuchtungsvorrichtung 24 und damit die gesamte Deckenleuchte 16 an der Unterkonstruktion 13 zum Beispiel mittels Schraubverbindungen befestigt und so in der abgehängten Decke 12 montiert werden.

Die Deckenleuchte 16 weist zudem eine Luftreinigungsvorrichtung 30 auf. Wie in Fig. 2, 4 und 5 veranschaulicht, ist diese Luftreinigungsvorrichtung 30 im Wesentlichen auf der der Raumdecke 11 zugewandten Oberseite der Beleuchtungsvorrichtung 24 angeordnet.

Die Luftreinigungsvorrichtung 30 weist einen langgestreckten Strömungskasten 31 mit einer im Wesentlichen quadratischen oder rechteckigen Querschnittsform auf. Der Strömungskasten 31 definiert in seinem Innern einen Luftkanal 32.

Der Luftkanal 32 erstreckt sich im Wesentlichen geradlinig von einem Lufteinlass 33 an einer Stirnseite des Strömungskastens 31 zu einem Luftauslass 35 an der gegenüber liegenden Stirnseite des Strömungskastens 31. Der Lufteinlass 33 ist über ein erstes Luftleitelement 34 mit der ersten Öffnung 20 im ersten Luftleitelement 34 selbst oder im Leuchtengehäuse 24 verbunden und der Luftauslass 35 ist über ein zweites Luftleitelement 36 mit der zweiten Öffnung 22 im zweiten Luftleitelement 36 selbst oder im Leuchtengehäuse 24 verbunden. Das erste und das zweite Luftleitelement 34, 36 können im Wesentlichen baugleich sein.

Das erste und das zweite Luftleitelement 34, 36 können für verschiedene Deckenleuchten 16 unterschiedlich gestaltet bzw. angepasst werden, sodass ein einheitlicher Strömungskasten 31 über die variablen Luftleitelemente 34, 36 mit verschiedenen Leuchtengehäusen 25 bzw. Beleuchtungsvorrichtungen 24 kombiniert werden kann.

Im Strömungskasten 31 der Luftreinigungsvorrichtung 30 ist ein Gebläse 38 angeordnet, zum Beispiel in eine entsprechende Gebläseaufnahme 39 eingehängt. Dieses Gebläse 38 fördert einen Luftstrom 40 aus dem Raum 10 über die erste Öffnung 20 und das erste Luftleitelement 34 zum Lufteinlass 33 des Strömungskastens 31, weiter durch den Luftkanal 32 zum Luftauslass 35 des Strömungskastens, und von diesem durch das zweite Luftleitelement 36 und die zweite Öffnung 22 zurück in den Raum 10. Da die erste und die zweite Öffnung 20, 22 auf einander abgewandten Seiten des Leuchtengehäuses 25 und damit möglichst weit voneinander beabstandet angeordnet sind, kann eine Zirkulation der Raumluft erzielt werden, ohne dass der aus der zweiten Öffnung 22 ausgegebene Luftstrom 40 direkt wieder in die erste Öffnung 20 gesaugt wird.

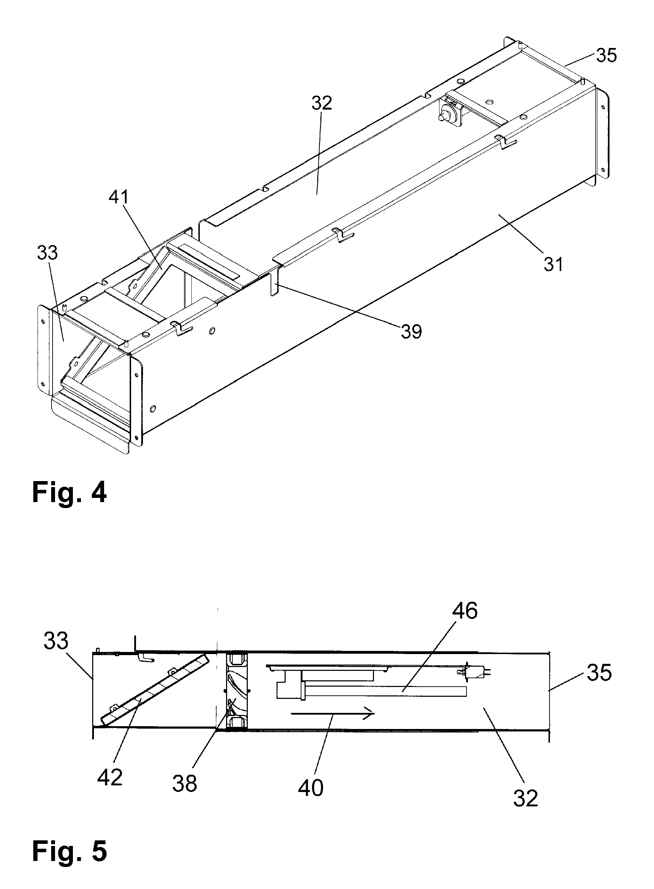

Stromauf des Gebläses 38 (links in Fig. 4 und 5) ist eine Filterhalterung 41 für wenigstens einen Luftfilter 42 in dem Strömungskasten 31 angeordnet. Die Filterhalterung 41 bzw. der wenigstens eine Luftfilter 42 sind bevorzugt schräg im Luftkanal 32 angeordnet (vgl. Fig. 4 und 5); alternativ können sie aber auch im Wesentlichen senkrecht zum Luftkanal 32 ausgerichtet sein. Der Luftfilter 42 soll Partikel und Substanzen aus dem Luftstrom 40 entfernen und so die Raumluft reinigen. Da der Luftfilter 42 stromauf des Gebläses 38 angeordnet ist, wird eine Verschmutzung des Gebläses 38 durch verunreinigte Raumluft verringert.

Der Luftfilter 42 kann vorzugsweise aus dem Strömungskasten 31 der Luftreinigungsvorrichtung 30 entfernt werden, um ihn zu reinigen oder gegen einen neuen Luftfilter auszutauschen. Alternativ (oder zusätzlich) kann ein solcher Luftfilter 42 auch in dem ersten Luftleitelement 34 oder in der ersten Öffnung 20 angeordnet sein.

In dem Strömungskasten 31 der Luftreinigungsvorrichtung 30 ist ferner eine UV-Strahlungseinrichtung 46 angeordnet. Diese UV-Strahlungseinrichtung 46 ist beispielsweise an einem Deckel 44 des Strömungskastens 31 angebracht und ragt in den Luftkanal 32 hinein.

Die UV-Strahlungseinrichtung 46 weist eine UV-Lampe auf, die insbesondere kurzwellige UV-C-Strahlung (100 - 280 nm) emittiert. Durch Bestrahlung des an der UV-Strahlungseinrichtung 46 vorbei geleiteten Luftstroms 40 mit dieser UV-C-Strahlung können Keime, Bakterien, Viren, Hefen, Schimmelpilzsporen, etc. im Luftstrom 40 abgetötet werden, wodurch die Raumluft desinfiziert wird.

Das Strömungsvolumen und die Strömungsgeschwindigkeit des durch das Gebläse 38 geförderten Luftstroms 40 und die Strahlungsleistung der UV-Strahlungseinrichtung 46 sind so aufeinander abgestimmt, dass eine gewünschte bzw. ausreichende Dosierung (Bestrahlungszeit × Bestrahlungsintensität) gewährleistet ist, um einen gewünschten bzw. ausreichenden Desinfektionsgrad der Raumluft zu erzielen. Die Öffnungen 20, 22, die Luftleitelemente 34, 36 und der Strömungsquerschnitt des Luftkanals 32 sind ebenfalls auf diese Vorgaben abgestimmt.

Vorzugsweise ist die Luftreinigungsvorrichtung 30 derart dimensioniert, dass in etwa die Luft jenes Raumbereichs gereinigt bzw. rein gehalten werden kann, der durch die Beleuchtungsvorrichtung 24 beleuchtet wird. Auf diese Weise kann erreicht werden, dass bei einer Bestückung der abgehängten Decke mit Deckenleuchten 16 zum Zwecke einer ausreichenden Raumbeleuchtung automatisch auch eine ausreichende Desinfektionswirkung für die Raumluft vorhanden ist.

In einer Ausführungsvariante für ein Kassettendeckenmaß von 620 mm × 620 mm hat auch der Rahmen 26 des Leuchtengehäuses 25 ein Außenmaß von etwa 620 mm × 620 mm und die Höhe des Leuchtengehäuses beträgt etwa 55 mm. Die Öffnungen 20, 22 haben jeweils eine Länge von etwa 200 mm und eine Breite von etwa 20 mm. Der Luftkanal 32 des Strömungskastens 31 hat eine Gesamtlänge von etwa 560 mm und einen quadratischen Querschnitt von etwa 90 mm × 90 mm. Diese Zahlenangaben sind lediglich beispielhaft, insbesondere ist die Erfindung nicht auf diese Maße und Maßkombinationen beschränkt.

Obwohl nicht dargestellt, ragen neben der UV-Lampe der UV-Strahlungs-einrichtung 46 auch zu kühlende (Leistungs-)Komponenten der Beleuchtungs-vorrichtung 24 (z.B. Steuerung, Kühlkörper, etc.) und der Luftreinigungsvorrichtung 30 (z.B. Kühlkörper, Steuerung, Gebläsemotor, etc.) in den Luftkanal 32, sodass diese Komponenten durch den Luftstrom 40 gekühlt werden können. Optional können weitere Kühlungsmaßnahmen vorgesehen sein.

Die Luftreinigungsvorrichtung 30 bzw. deren UV-Strahlungseinrichtung 46 ist bevorzugt unabhängig von der Beleuchtungsvorrichtung 24 bzw. deren wenigstens einem Leuchtmittel betreibbar. Auf diese Weise ist es möglich, je nach Bedarf die Luftreinigung und die Raumbeleuchtung gleichzeitig zu betreiben, nur die Luftreinigung durchzuführen oder nur die Raumbeleuchtung einzuschalten. Durch den bedarfsweise steuerbaren Betrieb der Komponenten 24, 30 der Deckenleuchte 16 kann Energie eingespart werden.

In einer Ausführungsvariante, die nicht dargestellt ist, trägt auch die UV-Strahlungseinrichtung 46 der Luftreinigungsvorrichtung 30 zur Raumbeleuchtung bei, insbesondere mit deren sichtbaren Strahlungsanteilen. Zu diesem Zweck ist die UV-Strahlungseinrichtung 46 mit einem Lichtaustritt zum Beispiel im Leuchtengehäuse 25 gekoppelt, um von der UV-Strahlungseinrichtung 46 erzeugte Strahlung in den Raum 10 auszusenden. Um das Aussenden schädlicher UV-Anteile in den Raum 10 zu verhindern, ist in dem Strahlengang ein entsprechender UV-Filter vorgesehen.

### BEZUGSZIFFERNLISTE

- 10: Raum
- 11: Raumdecke
- 12: abgehängte Decke
- 13: Unterkonstruktion
- 14: Deckenelement
- 16: Deckenleuchte
- 18: Leuchtfläche
- 20: erste Öffnung, erster Luftschlitz
- 22: zweite Öffnung, zweiter Luftschlitz
- 24: Beleuchtungsvorrichtung
- 25: Leuchtengehäuse
- 26: Befestigungseinrichtung
- 28: Ausnehmung für Leuchtmittel
- 30: Luftreinigungsvorrichtung
- 31: Strömungskasten
- 32: Luftkanal
- 33: Lufteinlass
- 34: erstes Luftleitelement
- 35: Luftauslass
- 36: zweites Luftleitelement
- 38: Gebläse, Lüfter
- 39: Gebläseaufnahme
- 40: Luftstrom
- 41: Filterhalterung
- 42: Luftfilter
- 44: Deckel
- 46: UV-Strahlungseinrichtung

## Patentansprüche

1. Deckenleuchte (16), aufweisend:
eine Beleuchtungsvorrichtung (24), welche ein Leuchtengehäuse (25) und wenigstens ein Leuchtmittel zum Emittieren von Licht im sichtbaren Wellenlängenbereich in dem Leuchtengehäuse aufweist;
eine Luftreinigungsvorrichtung (30), welche einen Strömungskasten (31) aufweist, welcher auf einer der Raumdecke (11) zugewandten Seite des Leuchtengehäuses (24) angeordnet ist und einen Luftkanal (32) darin definiert; und
eine Befestigungseinrichtung (26) zum Montieren der Beleuchtungsvorrichtung (24) und der Luftreinigungsvorrichtung (30) in einer von einer Raumdecke (11) eines Raums (10) abgehängten Decke (12),
wobei ein Lufteinlass (33) des Strömungskastens (31) über eine erste Öffnung (20) mit dem Raum (10) in Verbindung steht und ein Luftauslass (35) des Strömungskastens (31) über eine zweite Öffnung (22) mit dem Raum (10) in Verbindung steht,
wobei in dem Strömungskasten (31) ein Gebläse (38) angeordnet ist, um Luft vom Lufteinlass (33) durch den Luftkanal (32) hindurch zum Luftauslass (35) des Strömungskastens (31) zu fördern, und
wobei die Luftreinigungsvorrichtung (30) eine UV-Strahlungseinrichtung (46) aufweist, welche ausgestaltet ist, um die durch den Luftkanal (32) des Strömungskastens (31) hindurch strömende Luft (40) mittels UV-Strahlung zu reinigen.

2. Deckenleuchte nach Anspruch 1,
**dadurch gekennzeichnet, dass**
die erste Öffnung (20) und/oder die zweite Öffnung (22) in Ausnehmungen des Leuchtengehäuses (24) positioniert oder in dem Leuchtengehäuse (24) ausgebildet sind.

3. Deckenleuchte nach Anspruch 2,
**dadurch gekennzeichnet, dass**
der Lufteinlass (33) des Strömungskastens (31) mit der ersten Öffnung (20) über ein erstes Luftleitelement (34) verbunden ist und/oder der Luftauslass (35) des Strömungskastens (31) mit der zweiten Öffnung (22) über ein zweites Luftleitelement (36) verbunden ist.

4. Deckenleuchte nach einem der vorhergehenden Ansprüche,
**dadurch gekennzeichnet, dass**
im Luftkanal (32) des Strömungskastens (31) zwischen dem Lufteinlass (33) und dem Luftauslass (35) ein Luftfilter (42) angeordnet ist.

5. Deckenleuchte nach einem der vorhergehenden Ansprüche,
**dadurch gekennzeichnet, dass**
die Luftreinigungsvorrichtung (30) unabhängig von der Beleuchtungsvorrichtung (24) betreibbar ist.

6. Deckenleuchte nach einem der vorhergehenden Ansprüche,
**dadurch gekennzeichnet, dass**
die Beleuchtungsvorrichtung (24) einen Kühlkörper und/oder eine Steuervorrichtung aufweist, welcher/welche zumindest teilweise im Luftkanal (32) des Strömungskastens (31) angeordnet ist/sind.

7. Deckenleuchte nach einem der vorhergehenden Ansprüche,
**dadurch gekennzeichnet, dass**
die Luftreinigungsvorrichtung (30) einen Kühlkörper und/oder eine Steuervorrichtung und/oder einen Gebläsemotor aufweist, welcher/welche zumindest teilweise im Luftkanal (32) des Strömungskastens (31) angeordnet ist/sind.

8. Deckenleuchte nach einem der vorhergehenden Ansprüche,
**dadurch gekennzeichnet, dass**
die Befestigungseinrichtung (26) integral mit dem Leuchtengehäuse (25) der Beleuchtungsvorrichtung (24) oder dem Strömungskasten (31) der Luftreinigungsvorrichtung (30) ausgebildet ist.

9. Deckenleuchte nach einem der vorhergehenden Ansprüche,
**dadurch gekennzeichnet, dass**
die UV-Strahlungseinrichtung (46) mit einem Lichtaustritt gekoppelt ist, um von der UV-Strahlungseinrichtung (46) erzeugte Strahlung in den Raum (10) auszusenden.

10. Deckenleuchte nach einem der vorhergehenden Ansprüche,
**dadurch gekennzeichnet, dass**
das wenigstens eine Leuchtmittel der Beleuchtungsvorrichtung (24) wenigstens eine Leuchtiode oder Leuchtdiodengruppe aufweist.
